# EUROPEAN PATENT APPLICATION

(11) **EP 2 133 066 A1**
(43) Date of publication of application: **16.12.2009**
(21) Application number: 08158221.5
(22) Date of filing: 13.06.2008
(51) Int. Cl.: A61K 8/67, A61Q 19/08, A61Q 19/00, A61P 17/10

(54) **Cosmetic and dermatological composition containing a mixture of retinol derivatives and tocotrienols.**

(71) Applicant: Antonio Puig, S.A., 08021 Barcelona (ES)
(72) Inventor: Panyella Costa, David, 08912, Badalona (Barcelona) (ES); Ginestar Gonzàlez, José, 08320 El Masnou (Barcelona) (ES); Balaguer Sancho, Francisco, 08024, Barcelona (ES); Recasens, Mar, 08005, Barcelona (ES)
(74) Representative: Barlocci, Anna

(57) **Abstract**

The invention relates to a cosmetic or dermatological composition consisting of the following components: (i) a safe and effective amount of a mixture consisting of two ore more unsaturated C18 fatty acid esters of retinol and, optionally, one or more saturated C16-C18 fatty acid ester of retinol; (ii) a safe and effective amount of one or more tocotrienols; (iii) optionally, a safe and effective amount of one or more tocopherols; and (iv) one or more cosmetically or dermatologically acceptable carriers, wherein the components (i), (ii) and (iii) optionally include either non active components or non effective amounts of other components. The composition provides an improvement of the cosmetic appearance of the skin that is higher than expected due to a unexpected synergistic effect. It is also useful for the treatment of dermatological disorders.

## Description

The present invention relates to a cosmetic composition containing mixtures of long chain fatty acid esters of retinol and tocotrienols, and to their use for skin care treatment.

### BACKGROUND ART

Skin is subject to deterioration through intrinsic aging (chronoaging) and environmental factors, primarly photaging, being distinct differences between chrono and photoaging (cf. EP0379367). Skin care compositions containing retinoids for treating photo damaged skin and/ or intrinsically aged skin are quite prominent.

Retinol (Vitamin A) and synthetic derivatives have been used extensively in the treatment of skin disorders and have been used as skin repair or renewal agents, alone or in combination with other active ingredients.

The retinoid-like activity of fatty acid esters of retinol are considered lower than retinol but have been successfully used to treat dermatological and chronoaging conditions without skin irritation.

EP-A-807429 describes the use of long chain (C18-C30) unsaturated fatty acid esters of retinol for enhancing skin radiance without substantial irritation and for treating chronoaging conditions and dermatological disorders.

It is known that some retinoids derivatives quickly lose their activity and either oxidize or isomerize to non-efficacious chemical forms with the result that the amount of retinoid actually available to provide the beneficial effects of the product is reduced. There have been several attempts to formulate a stable composition comprising retinoid derivatives. As an instance, WO 9607396 addresses the preference of certain retinyl esters, retinol and retinal as preferred forms in formulating stable skin care compositions with naturally occurring retinoids.

On the other hand, it is also known that the antioxidant activity of tocotrienols prevents free radical damage to cells and cell components, and so they may help prevent skin aging due to damage from free radicals generated by UV rays and environmental pollutants. The protective effects of tocotrienols against oxidation helps protect skin cells from the impact of aging. EP-A-801565 discloses the use of tocotrienols in the substantial absence of tocopherols for topical application to the skin or hair for the purpose of improving the cosmetic appearance of the skin or hair.

Vitamin E is a generic term for compounds that qualitatively exhibit the biological activity of alpha-tocopherol. The eight naturally occurring vitamin E compounds are alpha-, beta-, gamma-, delta-tocopherols and alpha-, beta-, gamma-, delta-tocotrienols. The main biological activity of vitamin E is believed to be due to its antioxidant function and it has been widely used in dermatological compositions as an antioxidant. Non-antioxidant roles of vitamin E members have also been described (cf. C. Debier et al., "Vitamins A and E: metabolism, roles and transfer to offspring", Brit. J. Nutr. 2005, vol. 93, pp. 153-174).

While various agents have heretofore been provided for dermatological conditions, there continues to be a need for effective cosmetic compositions for topical application to skin providing superior benefits in treating or preventing the signs of aging and photodamaged skin.

### SUMMARY OF THE INVENTION

Although it is known that fatty acid esters of retinol which are both unsaturated and long chain (C18-C30) are useful for skin care treatment for chronoaging conditions and dermatologic disorders and that the protective effects of tocotrienols against oxidation helps protect skin cells from the impact of aging, the combined use of a mixture of unsaturated C18 fatty acid esters of retinol,̅ and one or more tocotrienols, optionally, together with one or more tocopherols, in a cosmetic composition as the only active ingredients has never been suggested. The inventors have surprisingly found that the combination of the mentioned active ingredients in a cosmetic composition provides an improvement of the cosmetic appearance of the skin that is higher than expected due to a synergistic effect.

Furthermore, the specific combination of unsaturated C18 fatty acid esters of retinol and one or more tocotrienols unexpectedly provides an stabilizing effect to the composition of the invention, which retains its activity during storage, even if one or more saturated C16-C18 fatty acid ester of retinol, such as retinyl palmitate, and retinyl stearate are added.

Thus, one aspect of the invention is related to a cosmetic or dermatological composition consisting of the following components: (i) a safe and effective amount of a mixture consisting of two or more unsaturated C18 fatty acid esters of retinol and, optionally, one or more saturated C16-C18 fatty acid esters of retinol; (ii) a safe and effective amount of one or more tocotrienols; (iii) optionally, a safe and effective amount of one or more tocopherols; and (iv) one or more cosmetically or dermatologically acceptable carrier, wherein the components (i), (ii) and (iii) optionally include either non active components or non effective amounts of other components.

The cosmetic composition of the invention can be used for topical application to the skin for the purpose of improving the cosmetic appearance of the skin without substantial irritation. Thus, another aspect of the present invention is the use of the cosmetic composition as defined above for skin care treatment for chronoaging conditions, for repairing photodamaged skin, to reduce or prevent the damaging effects of ultra-violet light on skin, or for providing cosmetic skin care benefits comprising treating/preventing boosting collagen deposition in skin, enhancing tissue repair, improving skin texture, improving smoothness and firmness, and lightening skin. Chronoaging conditions includes, for instance, wrinkles and fine lines, leatheriness, yellowing, sagging, mottling (hyperpigmentation) and age spots.

Also forms part of the invention a cosmetic method for improving the cosmetic appearance of the skin without substantial irritation, the methods comprising topically applying to the skin the composition of the invention with the aid of iontophoretic patches or mechanical massaging devices.

The composition is also useful for the treatment of dermatological disorders. Therefore, a dermatological composition as defined above for the treatment of a dermatological disorder selected from acne, follicular and lesional papules, actinic keratoses, oily skin and rosacea is part of the invention. So, the use of a combination of (i) a safe and effective amount of a mixture consisting of two or more unsaturated C18 fatty acid esters of retinol and, optionally, one or more saturated C16-C18 fatty acid esters of retinol; (ii) a safe and effective amount of one or more tocotrienols; (iii) optionally, a safe and effective amount of one or more tocopherols; and (iv) one or more cosmetically or dermatologically acceptable carriers, wherein the components (i), (ii) and (iii) optionally include either non active components or non effective amounts of other components, for the manufacture of a medicament for the treatment of a dermatological disorder as defined above is also part of the invention.

The invention also relates to a method of preventive and/or therapeutic treatment of a mammal, including a human, which is capable of suffering or is suffering from a dermatological disorder selected from acne, follicular and lesional papules, actinic keratoses, oily skin and rosacea. This method comprises the administration to said patient of the composition of the invention with or without the aid of iontophoretic patches or mechanical massaging devices.

Still another aspect of the invention relates to a kit for the application of the cosmetic composition of the invention, the kit comprising: (i) said cosmetic composition, and (ii) an iontophoretic patch, the patch comprising an electrical power source and electrodes optionally covered by an hydrogel.

Still another aspect of the invention relates to a kit for the application of the cosmetic composition of the invention, the kit comprising: (i) said cosmetic composition, and (ii) a massaging device.

### DETAILED DESCRIPTION OF THE INVENTION

"Safe and effective amounts" of unsaturated C18 fatty acid esters of retinol, tocotrienols and, optionally, of saturated C16-C18 fatty acid ester of retinol, and of tocopherols are to be used within cosmetic compositions of the present invention. The term "safe and effective amounts" is defined as any amount sufficient to significantly improving the cosmetic appearance of the skin without substantial irritation, but low enough to avoid serious side effects (at a reasonable benefit/risk ratio), within the scope of sound medical judgement.

The safe and effective amount of the esters, the tocotrienols and/or the tocopherols will vary with the age and physical condition of the consumer, the condition of the skin, the duration of the treatment, the nature of any concurrent treatment, the specific combination of active ingredients employed, the particular cosmetically acceptable carrier utilized, and like factors in the knowledge and expertise of any attending physician.

In a preferred embodiment of the composition of the invention, the weight ratio of the esters of retinol (component (i)) to the one or more tocotrienols (component (ii)) and, optionally, the one or more tocopherols (component (iii)) is from 1:10 to 10:1. Preferably, the mentioned ratio is from 1:2 to 6:1, more preferably, the mentioned ratio is 1:1. As it is shown in the examples, specially good activity results are obtained with this mixture of components.

In another preferred embodiment of the composition of the present invention, unsaturated C18 fatty acid esters of retinol are selected from the group consisting of retinyl oleate, retinyl linoleate, retinyl linolenate, and isomers thereof. More preferably, the mixture of unsaturated C18 fatty acid esters of retinol and, optionally, one or more saturated C16-C18 fatty acid ester of retinol is a mixture of retinyl palmitate, retinyl stearate, retinyl oleate, retinyl linoleate, and retinyl linolenate. Fatty acid esters of retinol, when obtained from natural sources, can be accompained by non effective amounts of other components.

In another preferred embodiment, in the mixture of fatty acid esters of retinol the amount of retinyl palmitate may range from 10% by weight to 25% by weight, the amount of retinyl stearate may range from 1% by weight to 5% by weight, the amount of retinyl oleate may range from 20% by weight to 50% by weight, the amount of retinyl linoleate may range from 20% by weight to 50% by weight, and the amount of retinyl linolenate may range from 1 % by weight to 5% by weight of said mixture, provided that the total percentage of components is 100%.

In a still more preferred embodiment, the mixture of fatty acid esters of retinol is obtained from rice bran oil, namely the amount of retinyl palmitate may range from 15% by weight to 20% by weight, the amount of retinyl stearate may range from 1 % by weight to 2% by weight, the amount of retinyl oleate may range from 30% by weight to 40% by weight, the amount of retinyl linoleate may range from 30% by weight to 40% by weight, and the amount of retinyl linolenate may range from 1 % by weight to 2% by weight of said mixture, provided that the total percentage of components is 100%.

In another preferred embodiment of the composition of the present invention the amount of the mixture of fatty acid esters of retinol is from 0.001 % to 10% by weight. Preferably, the amount of the mixture of esters of retinol is form 0.01 % to 1% by weight, more preferably, from 0.01 to 0.5% by weight.

Tocotrienols, together with tocopherols, compose the vitamin E family. As used herein, the term "tocotrienol" encompasses Vitamin E derivatives bearing unsaturated hydrocarbon tails, having the following general formula:

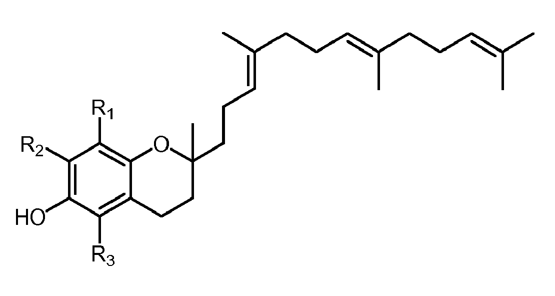

| Type | R1 | R2 | R3 |
|---|---|---|---|
| alpha-tocotrienol | Me | Me | Me |
| beta-tocotrienol | Me | H | Me |
| gamma-tocotrienol | Me | Me | H |
| delta-tocotrienol | Me | H | H |

including, but not limited to, alpha-, beta-, gamma-, delta-tocotrienols, and mixtures thereof. The double bonds may be cis or trans or mixtures thereof. The term "tocopherol" encompasses their counterparts bearing saturated tails, including, but not limited to, alpha-, beta-gamma-, and delta-tocopherols, and mixtures thereof. All the tocotrienol and tocopherol isomers are important antioxidants due to the ease of donating a hydrogen atom from the hydroxyl group on the chromanol ring to reduce free radicals. Both tocotrienols and tocopherols may be either synthetic or naturally occurring ones obtained, for example, from cereal grains (ie. oat, barley, and rye), coconut oil, palm oil, rice bran oil, and soybean oil.

In another preferred embodiment of the composition of the present invention, the tocotrienol is selected from the group consisting of alpha-tocotrienol, beta-tocotrienol, gamma-tocotrienol, delta-tocotrienol, and mixtures thereof. Preferably the tocotrienol is gamma-tocotrienol.

In another preferred embodiment of the composition of the present invention, the amount of the one or more tocotrienols, optionally, in admixture of the one or more tocopherols is 0.001% to 10% by weight. Preferably, this amount is from 0.01% to 1% by weight, more preferably, from 0.01 to 0.5% by weight.

When obtained from natural materials, the extract resulted therefrom is a mixture of alpha, beta, gamma, and delta-tocotrienol (hereinafter, referred to as "total tocotrienol") and alpha, beta, gamma, delta-tocopherols (hereinafter, referred to as the "total tocopherol") and other oil non-active components derived from the raw material. As such extract, there may be preferably used commercially available products, for example, "NuTriene tocotrienol" (30% content, a trade name, a product of Eastman Chemical Company), "Oryzatocotrienol" (a trade name, a product of Oryza Oil & Fat Chemical Co., Ltd.), "Tocotrienol oil" (70% content, a product of Golden Hope Plantations Berhad), and "Tocotrienol oil" (30% content, 50% content, a product of Carotech Sdn Bhd.).

For example, Nutriene tocotrienol is a mixture consisting of from 4 to 10% by weight of alpha-tocotrienol, from 3 to 10% by weight of gamma-tocotrienol, from 10 to 13% by weight of alpha-tocopherol, from 2 to 5% by weight of gamma-tocopherol, from 2 to 15% by weight of other tocopherols and tocotrienols, and oil non-active components until completing 100% wt, being the content of the total tocotrienol at least a 15% by weight, the content of the total tocopherol from 13 to 18% by weight, and the combined content of the total tocotrienol and tocopherol of 30% by weight or more.

In another preferred embodiment of the composition of the present invention, the mixture of component (ii) and component (iii) is "NuTriene tocotrienol".

The term "cosmetically acceptable" or "dermatologically acceptable", as used herein, means that the compositions or components thereof so-described are suitable for use in contact with human skin without undue toxicity, incompatibility, instability, allergic response, or the like.

As used herein, the term "cosmetically acceptable carrier" or, "dermatologically acceptable carrier" includes any and all solvents, dispersion media, coatings, antibacterial and antifungal agents, isotonic and absorption delaying agents, and the like. The use of such media and agents for cosmetically active substances is well known in the art. Except insofar as any conventional media or agent is incompatible with the active ingredient, its use in the cosmetic compositions is contemplated.

Except in the operating and comparative examples, or where otherwise explicitly indicated, all numbers in this description indicating amounts, ranges or ratios of material are to be understood as modified by the word "about" and, consequently, should be considered approximate, unless specifically stated. All amounts are by weight of the final composition, unless otherwise specified.

The cosmetic or dermatological composition of the present invention can be formulated in several forms that include, but are not limited to, lotions, creams, gels, sticks, sprays, shaving creams, ointments, cleansing liquid washes and solid bars, shampoos, pastes, aerosol and non-aerosol sprays, powders, mousses, wipes, patches, nail lacquers, wound dressing and adhesive bandages, hydrogels, films, roll-ons, and make-up such as foundations, mascaras, and lipsticks.

The preferred composition may be in the form of a conventional skin-care product, such as, a cream, a gel or a lotion. Most preferably the product is a leave-on product, namely; a product to be applied to the skin without a deliberate rinsing step soon after its application to the skin. The composition can also be used in the form of a wash-off or rinse-off product .

The composition of the present invention also comprises a cosmetically or dermatologically acceptable topical carriers, including, but not limited to solutions, emulsions (e.g., microemulsions and nanoemulsions), gels, solids and liposomes. Other carriers can be formulated by those of ordinary skill in the art. Collectively water, solvents, silicones, esters, fatty acids, humectants and thickeners, or mixtures thereof, will constitute the cosmetically acceptable carrier in an amount from 1 % to 99.9% by weight, preferably from 80% to 99% by weight.

The composition of the present invention can be formulated as anhydrous products. "Anhydrous" shall mean that the composition contain less than about 10 % of water.

Alternatively, the composition of the invention can be formulated as a solution. Solutions typically include water (e.g., from 50% to 99.99% or from 90% to 99% of water). The composition of the present invention can be formulated as a solution comprising one or more emollient. Such compositions can contain from 2% to 50% of the one or more emollient. Emollients may be classified under such general chemical categories as esters, fatty acids, alcohols, polyols and hydrocarbons. A wide variety of suitable emollients are known and may be used herein. The International Cosmetic Ingredient Dictionary and Handbook (Gottschalck, Tara E. ; McEwen, GN (ed). 11th ed. Washington, DC: The Cosmetic, Toiletry and Fragrance Association, 2006.) contain numerous examples of suitable materials.

From such a solution, other products can be formulated, such as a lotion, a cream and an ointment. A lotion typically comprises from 1% to 20% by weight of one or more emollient and from 50% to 90% by weight of water. A cream typically comprises from 5% to 50% of one or more emollient and from 45% to 85% by weight of water. An ointment may comprise a simple base of animal or vegetable oils or synthetic hydrocarbons. An ointment may comprise from 2% to 10% by weight of one or more emollient plus from 0.1 % to 2% of one or more thickening agent or one or more viscosity increasing agents.

The compositon of the present invention can be formulated as an emulsion. In such a case, the carrier comprises from 1 % to 10% of one or more emulsifier. Emulsifiers may be non-ionic, anionic or cationic. Lotions and creams can be formulated as emulsions. Typically such lotions comprise from 0.5% to 5% by weight of one or more emulsifier. Such creams would typically comprise from 1 % to 20% of one or more emollient; from 20% to 80% by weight of water; and from 1 % to 10% by weight of an emulsifier. Single emulsion skin care preparations, such as lotions and creams, of the water-in-oil (W/O) type and the oil-in-water (O/W) type are well-known in the cosmetic art and are useful in the present invention. Multiphase emulsion compositions, for example of the water-in-oil-in-water (W/O/W) type or oil-in-water-in-oil (O/W/O) type are also useful in the present invention.

The compositions of the present invention can also be formulated as a gel (e.g. an aqueous gel using one or more suitable gelling agent and/or one or more solubilizing and delivery agents).

The compositions of the invention can also be formulated into a solid formulation (e.g. a wax-based stick, a wipe or a hydrogel patch).

Liposomal formulations and solid-lipid- nanoparticles are also useful compositions of the invention.

The compositions of the present invention may contain, in addition to the aforementioned components, a wide variety of additional oil-soluble materials and/or water-soluble materials, conventionally used in compositions for use on skin at their art-established levels.

Preferably, the composition of the present invention further includes one or more sunscreen agent to further lower skin's exposure to harmful UV rays.

As mentioned above, the composition of the invention may be advantageously applied by iontophoresis in combination with an iontophoretic patch.

Iontophoresis creates a potential gradient through the skin layers following the application of electrical current or voltage and inducing increased migration of ionic active agents into the skin by electrostatic repulsion at the active electrode: negative ions are delivered by the cathode and positive ions by the anode. In practice, the active agent, in an appropriate carrier (solution, gel or cream) is placed between the active electrode (also called the "delivery electrode") and the skin. The other electrode (also called the "return electrode") is placed elsewhere on the body. A mild electrical current is applied for a set time period.

An iontophoretic patch is a patch comprising an electrical power source (e.g., battery) and electrodes, optionally covered by an hydrogel. Just before their application on the area to be treated, a cosmetic formulation is smeared on the dry area of the patch. Preferably, patches are leaved on the area to be treated for about 20 minutes. Preferably, the voltage applied by the iontophoretic patch is comprised between 0.5 and 5 V. More preferably, the voltage is 3V.

As a consequence of the application of the composition of the invention in combination with the iontophoretic patch, a significant short-term effect on the improvement of the cosmetic appearance of the skin is noticed immediately after removal of the path.

Additionally, the composition of the invention may be advantageously applied with the help of a massager device.

In another embodiment, the topical compositions of this invention may be applied with a massaging device, such as a facial suction device for massaging. As a consequence of the application of the composition of the invention in combination with the massaging device the activity of the active ingredients is enhanced, and so a more effective facial treatment can be carried out comfortably.

Furthermore, the present invention covers all possible combinations of particular and preferred groups described hereinabove.

Throughout the description and claims the word "comprise" and variations of the word, such as "comprising", are not intended to exclude other additives, components, integers or steps. Additional objects, advantages and novel features of the invention will be set forth in part in the description, and in part will become apparent to those skilled in the art upon examination of the description or may be learned by practice of the invention.

### BRIEF DESCRIPTION OF THE DRAWINGS

Fig. 1 shows the % of viability (V) of the cells after treatment with the compositions defined in Example 9, versus non treated cells (NT).
Fig. 2 shows the % of increment (Δ) of the procollagen synthesis (PS) by human dermal fibroblasts treated with the compositons defined in Example 10.
Fig. 3 shows the % of viability (V) of the cells treated with the compositions defined in Example 9 and exposed to 5 J/cm² of UVA according to the procedure of Example 11. The measure was performed 24 hours after a second irradiation versus non irradiated and non treated cells (NT).

The following examples and drawings are provided by way of illustration, and are not intended to be limiting of the present invention.

### DETAILED DESCRIPTION OF PARTICULAR EMBODIMENTS

### EXAMPLE 1

The following composition of fatty acid esters of retinol, obtained from a rice bran oil:

| | | |
|---|---|---|
| Retinyl Ester Assay | 95.9% | HPLC area% |
| Retinyl Linoleate | 30.8% | HPLC area% |
| Retinyl Linolenate | 1.0% | HPLC area% |
| Retinyl Oleate | 40.0% | HPLC area% |
| Retinyl Palmitate | 19.7% | HPLC area% |
| Retinyl Stearate | 1.9% | HPLC area% |
| Free Fatty Acid | <1% | GC wt% |
| Free Retinol | <1% | HPLC area% |

was mixed with a mixture of tocotrienols and tocopherols commercialized under the trade name NuTriene® Tocotrienol, having the following composition.

| COMPONENT | mg/g |
|---|---|
| Alpha Tocotrienol | 67 |
| Gamma Tocotrienol | 82 |
| Beta Tocotrienol | <1 |
| Delta Tocotrienol | 5 |
| Total tocotrienols | 155 |
| Alpha Tocopherol | 115 |
| Gamma Tocopherol | 45 |
| Beta Tocopherol | 5 |
| Delta Tocopherol | 12 |
| Total Tocotrienols+ Tocopherols | 332 |

The weight ratio fo the mixture of esteres of retinol to NuTriene® Tocotrienol was 1:1. This compositon, from now on "composition of Example 1 ", was used as the active ingredient to prepare several cosmetic compositions.

### EXAMPLE 2

This example illustrates a protective moisturizing day face cream in accordance with the present invention.

| CHEMICAL | %w/w |
|---|---|
| AQUA | 45-55 |
| OCTOCRYLENE | 6-10 |
| CETEARYL ETHYLHEXANOATE | 2-7 |
| PROPYLENE GLYCOL STEARATE | 2-6 |
| BUTYL METHOXYDIBENZOYLMETHANE | 1-5 |
| ETHYLHEXYL SALICYLATE | 1-5 |
| CYCLOPENTASILOXANE | 1-5 |
| DIETHYLHEXYL BUTAMIDO TRIAZONE | 1-4 |
| GLYCERIN | 1-4 |
| ALUMINIUM STARCH OCTENYLSUCCINATE | 1-4 |
| BUTYLENE GLYCOL | 1-4 |
| DIMETHICONE | 1-5 |
| CETEARYL ALCOHOL | 0.5-2 |
| GLYCERYL STEARATE | 0.5-2 |
| PEG-100 STEARATE | 0.5-2 |
| 1-2 HEXANEDIOL | 0.2-0.6 |
| CAPRYLYL GLYCOL | 0.1-0.6 |
| CARBOMER | 0.2-0.8 |
| CICLOHEXASILOXANE | 0.1-0.75 |
| DIMETHICONE CROSSPOLYMER | 0.1-1 |
| DISODIUM EDTA | 0.1-0.5 |
| ETHYLHEXYLGLYCERIN | 0.25-0.75 |
| TROMETHAMINE | 0.1-1 |
| PARFUM | 0.05-0.7 |
| Composition of Example 1 | 0.01-2 |

### EXAMPLE 3

This example illustrates an anti-wrinkle face cream in accordance with the present invention.

| CHEMICAL | %w/w |
|---|---|
| AQUA | 58-70 |
| DIETHYLHEXYL CARBONATE | 3-7% |
| DIMETHICONE | 3-7 |
| GLYCERIN | 2.5-6 |
| ETHYLHEXYL PALMITATE | 1.5-3.5 |
| PROPYLENE GLYCOL DIPELARGONATE | 1.5-3 |
| HYDROXYETHYL ACRYLATE/SODIUM ACRYLOYLDIMETHYL TAURATE COPOLYMER | 1.5-3.5 |
| SQUALANE | 1-3 |
| CYCLOPENTASILOXANE | 1-3 |
| GLYCERYL STEARATE CITRATE | 1-3 |
| HYDROXYETHYLCELULOSE | 0.2-0.5 |
| 1-2 HEXANEDIOL | 0.2-0.7 |
| ALLUMINIUM STARCH OCTENYLSUCCINATE | 0.1-0.6 |
| BUTYLENE GLYCOL | 0.05-2 |
| CAPRYLYL GLYCOL | 0.2-0.7 |
| CYCLOHEXASILOXANE | 0.5-2 |
| DIMETHICONE CROSSPOLYMER | 0.2-1 |
| DIMETHICONOL | 0.1-0.6 |
| DISODIUM EDTA | 0.05-0.25 |
| ETHYLHEXYLGLYCERIN | 0.3-1 |
| GLYCERYL BEHENATE | 0.5-2 |
| HDI/TRIMETHYLOL HEXYLLACTONE CROSSPOLYMER | 0.5-2 |
| POLYGLYCERYL-2 DIPOLYHYDROXYSTEARATE | 0.3-1.5 |
| POLYSORBATE 60 | 0.2-08 |
| PVP | 0.1-0.5 |
| TRIETHANOLAMINE | 0.1-0.5 |
| MICA | 0.3-1 |
| PARFUM | 0.1-0.7 |
| Composition of Example 1 | 0.01-3 |

### EXAMPLE 4

This example illustrates an anti-wrinkle fase serum in accordance with the present invention.

| CHEMICAL | %w/w |
|---|---|
| AQUA | 70-90 |
| PROPYLENEGLYCOL | 2-8 |
| GLYCERIN | 2-7 |
| PEG-40 HYDROGENATED CASTOR OIL | 1-3 |
| MANNITOL | 0.5-3 |
| METHYLSILANOL MANNURONATE | 0.1-3 |
| ARGANIA SPINOSA KERNEL EXTRACT | 0.1-3 |
| SODIUM COCOYL GLUTAMATE | 0.1-3 |
| SODIUM HYALURONATE | 0.05-1 |
| 1-2 HEXANEDIOL | 0.2-0.6 |
| CAPRYLYLGLYCOL | 0.2-0.7 |
| CARBOMER | 0.15-0.5 |
| DISODIUM EDTA | 0.05-0.3 |
| ETHYLHEXYLGLYCERIN | 0.2-1 |
| TROMETHAMINE | 0.15-1 |
| PARFUM | 0.05-0.5 |
| PHENOXYETHANOL | 0.1-0.5 |
| Composition of Example 1 | 0.1-3 |

### EXAMPLE 5

This example illustrates an eye contour cream in accordance with the invention.

| CHEMICAL | %w/w |
|---|---|
| AQUA | 65-80 |
| ISOHEXADECANO | 2-8 |
| GLYCERIN | 2-6 |
| STEARETH 21 | 1-4 |
| BUTYLENE GLYCOL | 1-5 |
| CANOLA OIL | 1-4 |
| STEARETH-2 | 1-4 |
| 1,2 HEXANEDIOL | 0.1-0.6 |
| ACRYLATES C 10-30 ALKYL ACRYLATES CROSSPOLYMER | 0.1-0.6 |
| CAPRYLYL GLYCOL | 0.1-0.7 |
| CYCLOPENTASILOXANE | 0.5-3 |
| DISODIUM EDTA | 0.05-0.3 |
| HETHYLHEXYLGLYCERIN | 0.2-0.8 |
| MYRISTYL ALCOHOL | 0.5-2 |
| MYRISTYL GLUCOSIDE | 0.1-0.7 |
| TROMETHAMINE | 0.2-0.9 |
| Composition of Example 1 | 0.01-3 |

### EXAMPLE 6. Comparative example of an eye contour intensive treatment by combining an eye contour cream and an iontophoretic patch

An intensive treatment was carried out by applying the eye contour cream according to Example 5 comprising a 0.6% of the composition of Example 1 as the active ingredient in combination with an iontophoretic patch.

In the clinical assessment the results were compared with a blindly labelled and randomized control containing as the active ingredient a 0.6% of a 1:1 mixture of retinyl palmitate and of the NuTriene® Tocotrienol defined in Example 1, instead of the composition of Example 1. Fifty volunteers (female) aged 25- 45 years were recruited. Inclusion criteria for the tested subjects were healthy with no obvious skin disease and with fine wrinkles in the ocular area. They were requested not to use any cosmetic product in the evaluation zone at least three days before the evaluation day. The measurements were taken in an evaluation room with controlled temperature and relative humidity (20°C; 40-50% RH). Each volunteer was psychological and thermal accommodated for 15 minutes before each measurement. Each volunteer was provided with enough amount of product and patchs for one month of treatment and was instructed to use the contour eye cream twice every day (morning and night) and the ionic patch once a week. After one month the volunteers were called for the final measurements. All the measurements (initial and final) were performed in the same room, conditions and skin area.

### Biophysical measurements

Skin Hydration was measured with the Corneometer device (Courage Khazaka) as described in K. O'goshi et al., "Inter-instrumental variation of skin capacitance measured with the Corneometer®", Skin Res. Technol. 2005, vol. 11 (2), pp. 107 -109. Capacitance as a parameter for stratum corneum hydration is measured.

Transepidermal water loss (TEWL) was measured with the Tewameter device (Courage Khazaka) according to V. Rogiers an the EEMCO Group, "EEMCO guidance for the assessment of transepidermal water loss in cosmetic sciences", Skin Pharmacol. Appl. Skin Physiol. 2001, vol. 14(2), pp. 117-28. TEWL documents the integrity of stratum corneum barrier function. TEWL refers to the rate at which water migrates from the viable dermal tissues through the layers of the stratum corneum to the external environment.

Biomechanical properties were measured using the Cutometer device (Courage Khazaka) as described in G.E. Piérard and the EEMCO Group, "EEMCO Guidance to the in vivo Assessment of Tensile Functional Properties of the Skin. Part 1: Relevance to the Structures and Ageing of the Skin and Subcutaneous Tissues" Skin Pharmacol. Appl. Skin Physiol. 1999, vol. 12(6), pp. 352-362, and L. Rodrigues and the EEMCO Group, "EEMCO guidance to the in vivo assessment of tensile functional properties of the skin. Part 2: instrumentation and test modes", Skin Pharmacol. Appl. Skin Physiol. 2001, vol. 14(1), pp. 52-67. Review. The following parameters are measured: R₀: Extensibility has an implication for the firmness of the skin. This parameter represents the passive behavior of the skin to force; R₂: Ability of redeformation of the skin (gross elasticity). The closer the value is to 1 (100%) the more elastic the curve; R₅: Net elasticity. The closer the value is to 1 (100%) the more elastic the curve.

Skin wrinkles were measured using the Visiometer device (Courage Khazaka) as described in J.L. Leveque, "EEMCO guidance for the assessment of skin topography. The European Expert Group on Efficacy Measurement of Cosmetics and other Topical Products", J. Eur. Acad. Dermatol. Venereol. 1999, vol. 12(2), pp. 103-14. The following skin surface parameters were measured: Ra: arithmetic average roughness; and
Rz: average roughness.

Cutaneous ecographies were taken to measure the dermal density using Dermascan C (Cortex Technology) as described in H.K. Lee et al., "Comparison between ultrasonography (Dermascan C version 3) and transparency profilometry (Skin Visiometer SV600)", Skin Res. Technol. 2008, vol. 14(1), pp. 8-12. The echographic method enables an evaluation of physical properties of the skin. The different skin tissues induce variations on the acoustic behavior, which are expressed by a modification of the reflectivity to ultrasound.

The results of the biophysical measurements in volunteers treated with the eye contour cream of the invention in combination with an iontophoretic patch are shown in the following table:

**Table 1**

| | Initial | 1 month |
|---|---|---|
| Hydration (u.a.) | 51 (8) | 62* (6) |
| TEWL (g/m² h) | 13.72 (2.44) | 14.30 (2.46) |
| Ra (mm) ocular orbit area | 0.027 (0.005) | 0.023* (0.003) |
| Rz (mm) ocular orbit area | 0.181 (0.03) | 0.148* (0.02) |
| Extensibility R₀ (mm) | 0.4495 (0.0966) | 0.4303 (0.1035) |
| Elasticity R₂ (mm) | 0.6488 (0.1029) | 0.7180** (0.0912) |
| Elasticity R₅ (mm) | 0.3359 (0.0752) | 0.3870** (0.0756) |
| Increased skin density (%) | 28.92 (15.90) | |

| | | |
|---|---|---|
| *statistically significant difference respect initial value, Student's t-test (p<0.05) **statistically significant difference respect initial value, Student's t-test (p<0.1) | | |

Values between brackets indicate standard deviation.

The results show an increase of the cutaneous hydration, while maintaining the skin barrier integrity, an improvement of the biomechanical properties and an increase in dermal density as well as smoother roughness profile diminishing wrinkle depth.

As a consequence of the application of the eye contour cream in combination with the iontophoretic patch, a short-term effect on the reduction of the number and depth of wrinkles under the eye and the crow's feet area was noticed immediately after removal of the path. Additionally, a long term effect on skin topography was observed, with noticeable reduction of wrinkles depth. Clinical assessment during the treatment proved that fine wrinkles were substantially improved after 60 days of treatment. After treatment subjects were asked to self assess their skin condition and the results showed that the overall clarity and brightness of the skin were superior when the skin was treated with the inventive formula as opposed to the comparative samples.

### EXAMPLE 7. Comparative example using an anti-wrinkle face cream and a massager device

The anti-wrinkle face cream according to Example 3 was used comprising a 0.6% of the composition of Example 1. In the clinical assessment the results were compared with a blindly labelled and randomized control containing as the active ingredient a 0.6% of a 1:1 mixture of retinyl palmitate and of the NuTriene® Tocotrienol defined in Example 1, instead of the composition of Example 1. In the assay, the creams were applied with the help of a massager device.

A panel of 50 volunteers was selected ranging from 25-45 years old. The volunteers had fine and coarse wrinkles, overall photodamage and discrete and mottled pigmentation. Initial biophysical measurements were taken before treatment started in the ocular orbit area, the forehead and the nasogenian area in a temperature and humidity controlled environment. After 30 days of once-daily application (cream + massager device for 5 minutes) biophysical measurements were taken in 10 volunteers under the same initial areas and conditions.

The results of the biophysical measurements in volunteers treated with the anti-wrinkle face cream of the invention applied with the help of a massager device are shown in the following table:

**Table 2**

| | Initial | 1 month |
|---|---|---|
| Hydration (u.a.) | 57 (15) | 65 (12) |
| TEWL (g/m² h) | 13.76 (4.18) | 13.88 (2.98) |
| Rz (mm) ocular orbit area | 0.169 (0.03) | 0.142* (0.02) |
| Rz (mm) forehead | 0.129 (0.02) | 0.120 (0.02) |
| Rz (mm) nasogenian zone | 0.135 (0.026) | 0.115* (0.02) |
| Extensibility R₀ (mm) | 0.4116 (0.0531) | 0.3559** (0.0443) |
| Elasticity R₂ (mm) | 0.6290 (0.1005) | 0.7086** (0.1072) |
| Elasticity R₅ (mm) | 0.3344 (0.0927) | 0.3971** (0.0943) |
| Increased skin density (%) | 30.69 (19.51) | |

| | | |
|---|---|---|
| *statistically significant difference respect initial value, Student's t-test (p<0.05) **statistically significant difference respect initial value, Student's t-test (p<0.1) | | |

Values between brackets indicate standard deviation.

The results show an increase of the cutaneous hydration, maintaining the skin barrier integrity, an improvement of the biomechanical properties (elasticity and extensibility) and smoother roughness profile diminishing wrinkle depth.

Clinical assessment during the treatment proved that fine wrinkles were substantially improved after 60 days of treatment. Discrete pigmentation was improved after six months as well as overall photodamage. After treatment subjects were asked to self assess their skin condition and the results showed that the overall clarity and brightness of the skin were superior when the skin was treated with the inventive formula as opposed to the comparative samples.

### EXAMPLE 8. Comparative example using an anti-wrinkle face cream and an anti-wrinkle fase serum

An assay was carried out similarly as Example 7, but in addition to the application of the once-daily anti-wrinkle face cream defined therein, the anti-wrinkle fase serum according to Example 4 comprising a 0.6% of the composition of Example 1 was applied twice-daily. No massager device was used. Measurements were taken after 15 and 30 days.

In the clinical assessment the results were compared with the corresponding blindly labelled and randomized controls containing as the active ingredient a 0.6% of a 1:1 mixture of retinyl palmitate and of the NuTriene® Tocotrienol defined in Example 1, instead of the composition of Example 1.

The results of the biophysical measurements in volunteers treated with the anti-wrinkle face cream and the anti-wrinkle face serum of the invention are shown in the following table:

**Table 3**

| | Initial | 15 days | 1 month |
|---|---|---|---|
| Hydration (u.a.) | 40 (8) | 51* (9) | 59* (7) |
| TEWL (g/m² h) | 13.03 (3.70) | 14.01 (3.44) | 12.44 (3.04) |
| Ra (mm) ocular orbit area | 0.047 (0.007) | 0.035* (0.005) | 0.029* (0.006) |
| Ra (mm) forehead | 0.061 (0.016) | 0.052* (0.011) | 0.044* (0.014) |
| Ra (mm) nasogenian zone | 0.060 (0.019) | 0.045* (0.012) | 0.035* (0.008) |
| Rz (mm) ocular orbit area | 0.145 (0.028) | 0.115* (0.021) | 0.102* (0.026) |
| Rz (mm) forehead | 0.189 (0.029) | 0.160* (0.031) | 0.149* (0.024) |
| Rz (mm) nasogenian zone | 0.139 (0.021) | 0.123* (0.019) | 0.105* (0.017) |
| Extensibility R₀ (mm) | 0.360 (0.092) | 0.314 (0.053) | 0.267* (0.079) |
| Elasticity R₅ (mm) | 0.298 (0.094) | 0.323 (0.088) | 0.404* (0.112) |
| Increased skin density (%) | - | 26.9 (31.9) | 57.3 (48.2) |

| | | | |
|---|---|---|---|
| *statistically significant difference respect initial value, Student's t-test (p<0.05) | | | |

Values between brackets indicate standard deviation.

The results show an increase of the cutaneous hydration, maintaining the skin barrier integrity, an improvement of the biomechanical properties (extensibility and elasticity) and an increase in dermal density as well as smoother roughness profile diminishing wrinkle depth in every zone measured.

Clinical assessment during the treatment proved that fine wrinkles were substantially improved after 60 days of treatment. Discrete pigmentation was improved after six months as well as overall photodamage. After treatment subjects were asked to self assess their skin condition and the results showed that the overall clarity and brightness of the skin were superior when the skin was treated with the inventive formula as opposed to the comparative samples.

### EXAMPLE 9. Proliferation study

HaCaT keratinocyte cells (P. Boukamp et al. J. Cell. Biol. 1988, vol. 106, pp. 761-771; passages 5-25) were grown in Dulbecco's modified Eagle's medium (Sigma) supplemented with foetal bovine serum penicillin and streptomycin (Cambrex). HaCaT cells were seeded at 5000 cells/well in 24 well microplates and incubated at 37°C and 5% CO₂.

Cells cells were treated during 3 days with fresh medium containing 0.05 µl/ml Retinyl Palmitate (RP), 0.05 µl/ml composition of fatty acid esters of retinol of Example 1 (RO), 0.005 µl/ml NuTriene® Tocotrienol (T), and mixtures of RP with T (RP+T) and of RO with T (RO+T, namely, the composition of Example 1). At the end of the treatment viability was measured by Crystal Violet.

The evaluation of the statistic signification of the differences was performed by the Student's t-test for independent data (p<0.05).

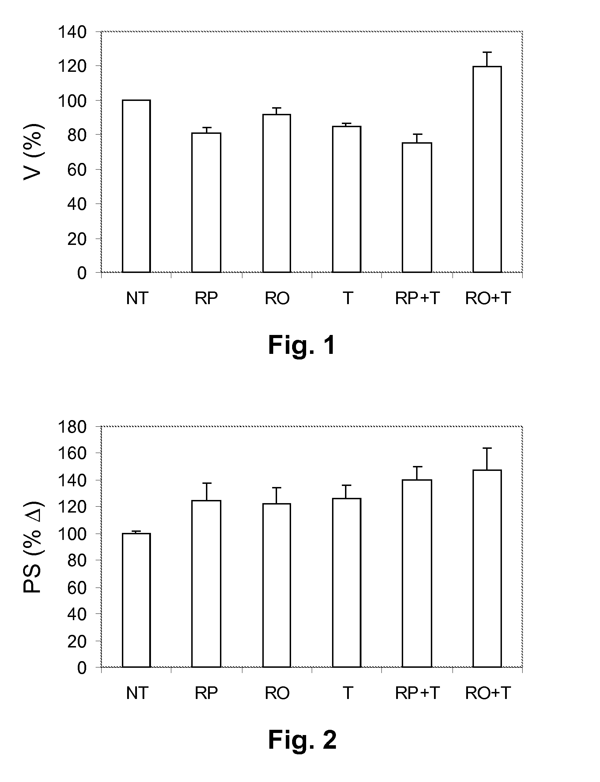

The results obtained of % of viability (V) of the cells versus non treated cells (NT) are shown in Table 4 and Fig.1.

**Table 4**

| Treatment Composition | Viability (%) |
|---|---|
| NT | 100,00 (3,23) |
| RP | 80,80 (3,75) |
| RO | 91,74 (2,30) |
| T | 84,39 (5,56) |
| RP+T | 74,89 (8,68) |
| RO+T | 119,48 (5,94) |

Values between brackets indicate standard deviation.

From Table 4, an increase on the viability provided by the fresh medium containing the mixture of RO and T is observed that is higher than expected according to the viability provided by the mediums containing each one of the active ingredients separately. So, the results show that the combination of the composition of fatty acid esters of retinol of Example 1 and the NuTriene® Tocotrienol, namely the composition of Example 1, provides a synergistic effect on the viability of cells treated with a composition containing one of these components as the only active ingredient.

### EXAMPLE 10. Collagen synthesis study

Human dermal fibroblasts were obtained as primary cultures. Fibroblasts (J.Varani et al. Am. J. Pathol. 2001, vol. 158, pp. 931-942; passages 1-7) were grown in Dulbecco's modified Eagle's medium (Sigma) supplemented with foetal bovine serum penicillin and streptomycin (Cambrex). Fibroblast were seeded at 5000 cells/well in 24 well microplates and incubated for 37°C and 5% CO², in a culture medium with low concentration Ca²⁺ (0.15 mM).

Cells were treated during 6 days with fresh medium containing 0.05 µl/ml Retinyl Palmitate (RP), 0.05 µl/ml composition of fatty acid esters of retinol of Example 1 (RO), 0.005 µl/ml NuTriene® Tocotrienol (T), and mixtures of RP with T (RP+T) and of RO with T (RO+T, namely the composition of Example 1). The culture medium was used in the evaluation of Procollagen Type I by ELISA (Takara). Results are expressed as % of increase.

The evaluation of the statistic signification of the differences was performed by the Student's t-test for independent data (p<0.06).

The results obtained of the % of increment (Δ) of the procollagen synthesis (PS) by human dermal fibroblasts versus non treated cells (NT) are shown in Table 5 and Fig. 2.

**Table 5**

| Treatment Composition | PS (%Δ) |
|---|---|
| NT | 100,00 (1,32) |
| RP | 124,44 (12,67) |
| RO | 121,79 (12,72) |
| T | 126,34 (9,59) |
| RP+T | 140,10 (9,83) |
| RO+T | 146,93 (17,10) |

Values between brackets indicate standard deviation.

The results from Table 5 reveal an increase of the % of procollagen synthesis after treatment with fresh mediums containing RP+T and RO+T. The increase of collagen synthesis after treatment with fresh medium containing RO+T is higher than expected according to the results obtained by the mediums containing each one of the active ingredients separately. So, the results show that the combination of the composition of fatty acid esters of retinol of Example 1 and the NuTriene® Tocotrienol, namely the composition of Example 1, provides a synergistic effect on the collagen synthesis of cells treated with a composition containing one of these components as the only active ingredients.

### EXAMPLE 11. UVA protection study

Human dermal fibroblasts were obtained as primary cultures. Fibroblasts (J.Varani et al. Am. J. Pathol. 2001, vol. 158, pp. 931-942; passages 1-7) were grown in Dulbecco's modified Eagle's medium (Sigma) supplemented with foetal bovine serum penicillin and streptomycin (Cambrex). Fibroblast were seeded at 5000 cells/well in 24 well microplates and incubated for 37°C and 5% CO₂, in a culture medium with low concentration Ca²⁺ (0.15 mM).

Cells were treated during 7 days with fresh medium containing 0.02 µl/ml RP, 0.02 µl/ml RO, 0.002 µl/ml T, and mixtures RP+T and RO+T. At day 4 and 6 human dermal fiborblastes were exposed to 5 J/cm² of UVA. Viability was measured by Crystal Violet 24 hours after last irradiation.

The evaluation of the statistic signification of the differences was performed by the Student's t-test for independent data (p<0.05)

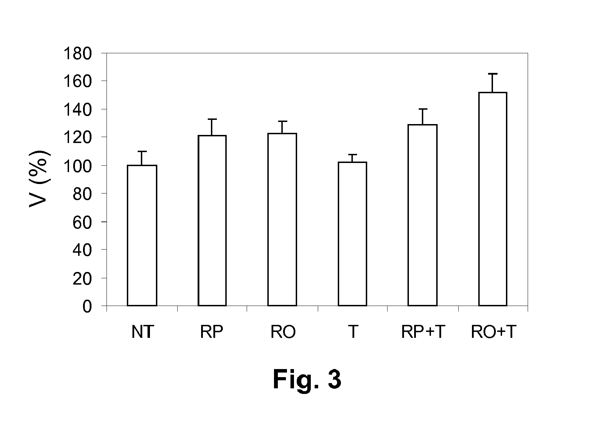

The results obtained are expresed as % of viability (V) of the cells versus non irradiated and non treated cells (NT) are shown in Table 6 and Fig.3.

**Table 6**

| Treatment Composition | Viability (%) |
|---|---|
| NT | 100.00 (9.8) |
| RP | 120.9 (11.6) |
| RO | 122.6 (9.0) |
| T | 102.0 (5.9) |
| RP+T | 128.8 (11.5) |
| RO+T | 151.9 (13.3) |

Values between brackets indicate standard deviation.

From Table 6, an increase on the UVA protection expresed as % of viability provided by the fresh medium containing the mixture of RO and T is observed that is higher than expected according to the UVA protection provided by the mediums containing each one of the active ingredients separately. So, the results show that the combination of the composition of fatty acid esters of retinol of Example 1 and the NuTriene® Tocotrienol, namely the composition of Example 1, provides a synergistic effect on the UVA protection of cells treated with a composition containing one of these components as the only active ingredient.

## Claims

1. A cosmetic or dermatological composition consisting of the following components:
(i) a safe and effective amount of a mixture consisting of two or more unsaturated C18 fatty acid esters of retinol and, optionally, one or more saturated C16-C18 fatty acid esters of retinol;
(ii) a safe and effective amount of one or more tocotrienols;
(iii) optionally, a safe and effective amount of one or more tocopherols; and
(iv) one or more cosmetically or dermatologically acceptable carriers,
wherein the components (i), (ii) and (iii) optionally include either non active components or non effective amounts of other components.

2. The composition according to claim 1, wherein the weight ratio of the esters of retinol to the one or more tocotrienols and, optionally, one or more tocopherols is from 1:10 to 10:1.

3. The composition according to any one of claims 1 or 2, wherein the unsaturated C18 fatty acid ester of retinol is selected from the group consisting of retinyl oleate, retinyl linoleate, retinyl linolenate, and isomers thereof.

4. The compositon according to any one of claims 1 to 3, wherein the amount of the mixture of fatty acid esters of retinol is from 0.001 % to 10% by weight.

5. The composition according to any one of claims 1 to 4, wherein the tocotrienol is selected from the group consisting of alpha-tocotrienol, beta-tocotrienol, gamma-tocotrienol, delta-tocotrienol, and mixtures thereof.

6. The composition according to claim 5, wherein said tocotrienol is gamma-tocotrienol.

7. The composition according to any one of claims 1 to 6, wherein the amount of the one or more tocotrienols, optionally, in admixture of the one or more tocopherols is 0.001 % to 10% by weight.

8. The composition according to any one of claims 1 to 7, wherein
the mixture of component (ii) and component (iii) consists of from 4 to 10% by weight of alpha-tocotrienol, from 3 to 10% by weight of gamma-tocotrienol, from 10 to 13% by weight of alpha-tocopherol, from 2 to 5% by weight of gamma-tocopherol, from 2 to 15% by weight of other tocopherols and tocotrienols, and oil non-active components until completing 100% wt, being the content of the total tocotrienol at least a 15% by weight, the content of the total tocopherol from 13 to 18% by weight, and the combined content of the total tocotrienol and tocopherol of 30% by weight or more.

9. The composition according to claims 1 to 8 further including one or more sunscreen agent.

10. Use of a cosmetic composition as defined in any one of claims 1 to 9, for skin care treatment for chronoaging conditions, for repairing photodamaged skin, to reduce or prevent the damaging effects of ultra-violet light on skin, or for providing cosmetic skin care benefits comprising treating/preventing boosting collagen deposition in skin, enhancing tissue repair, improving skin texture, improving smoothness and firmness, and lightening skin.

11. Use according to claim 10, wherein the chronoaging conditions is selected from the group consisting of wrinkles and fine lines, leatheriness, yellowing, sagging, mottling (hyperpigmentation), and age spots.

12. Dermatological composition as defined in any one of claims 1 to 9 for the treatment of a dermatological disorder selected from the group consisting of acne, follicular and lesional papules, actinic keratoses, oily skin and rosacea.

13. A kit for the application of the cosmetic composition as defined in any one of claims 1 to 9, the kit comprising:
(i) said cosmetic composition, and
(ii) an iontophoretic patch, the patch comprising an electrical power source and electrodes optionally covered by an hydrogel.

14. A kit for the application of the cosmetic composition as defined in any one of claims 1 to 9, the kit comprising:
(i) said cosmetic composition, and
(ii) a massaging device.
